# EUROPEAN PATENT SPECIFICATION

(11) **EP 1 767 214 B1**
(45) Date of publication and mention of the grant of the patent: **13.05.2009**
(21) Application number: 05425666.4
(22) Date of filing: 23.09.2005
(51) Int. Cl.: A61K 39/39, A61K 39/02, A61P 37/00

(54) **Use of neurotrophil activating protein of helicobacter pylori (HP-NAP) and/or of its portions as adjuvants for the development of T helper type 1 (TH1) immune responses**
Verwendung des Neutrophil-activating-protein von Helicobacter pylori und/oder Teile davon als Adjuvant für die Induktion einer T-helper type 1 (TH1) Immunantwort
Utilisation de protein d'activation des neutrophiles d'Helicobacter pylori (HP-NAP) et/ou de portions de celle-ci comme adjuvant pour l'obtention de réponses immunes par des lymphocytes T helper de type 1 (TH1).

(43) Date of publication of application: 28.03.2007
(73) Proprietor: Del Prete, Gianfranco, 50134 Firenze (IT); D'Elios, Mario Milco, 50053 Empoli FI (IT); Amedei, Amedeo, 50100 Firenze (IT); Cappon, Andrea, 35010 Padova (IT); de Bernard, Marina, 35141 Padova (IT)
(72) Inventor: Del Prete, Gianfranco, 50134 Firenze (IT); D'Elios, Mario Milco, 50053 Empoli FI (IT); Amedei, Amedeo, 50100 Firenze (IT); Cappon, Andrea, 35010 Padova (IT); de Bernard, Marina, 35141 Padova (IT)
(74) Representative: Bardini, Marco Luigi

(56) References cited:
- WO-A-01/00857
- WO-A-02/45741
- WO-A-99/53310
- WO-A-99/57278
- WO-A-03/018054
- MOINGEON P ET AL: "Towards the rational design of Th1 adjuvants" VACCINE, BUTTERWORTH SCIENTIFIC. GUILDFORD, GB, vol. 19, no. 31, 14 August 2001 (2001-08-14), pages 4363-4372, XP004296096 ISSN: 0264-410X
- DREDGE KEITH ET AL: "Adjuvants and the promotion of Th1-type cytokines in tumour immunotherapy." CANCER IMMUNOLOGY IMMUNOTHERAPY, vol. 51, no. 10, November 2002 (2002-11), pages 521-531, XP002376288 ISSN: 0340-7004
- MOINGEON P: "Strategies for designing vaccines eliciting Th1 responses in humans" JOURNAL OF BIOTECHNOLOGY, vol. 98, no. 2-3, 25 September 2002 (2002-09-25), pages 189-198, XP002376289 ISSN: 0168-1656
- DUNDON W G ET AL: "The neutrophil-activating protein of Helicobacter pylori" INTERNATIONAL JOURNAL OF MEDICAL MICROBIOLOGY, URBAN UND FISCHER, DE, vol. 291, no. 6-7, 2001, pages 545-550, XP004960091 ISSN: 1438-4221
- MONTECUCCO CESARE ET AL: "Molecular and cellular mechanisms of action of the vacuolating cytotoxin (VacA) and neutrophil-activating protein (HP-NAP) virulence factors of Helicobacter pylori." MICROBES AND INFECTION, vol. 5, no. 8, July 2003 (2003-07), pages 715-721, XP002376290 ISSN: 1286-4579
- D'ELIOS M M ET AL: "Helicobacter pylori, T cells and cytokines: the ''dangerous liaisons''" FEMS IMMUNOLOGY AND MEDICAL MICROBIOLOGY, ELSEVIER SCIENCE B.V., AMSTERDAM, NL, vol. 44, no. 2, 1 May 2005 (2005-05-01), pages 113-119, XP004869941 ISSN: 0928-8244
- PAN JIANPING ET AL: "Interferon-gamma is an autocrine mediator for dendritic cell maturation" IMMUNOLOGY LETTERS, vol. 94, no. 1-2, 15 June 2004 (2004-06-15), pages 141-151, XP002376291 ISSN: 0165-2478

### SEQUENCE LISTING

<110> Marina De Bernard, Andrea Cappon, Gianfranco Del Prete, Mario Milco D'Elios, Amedeo Amedei
<120> USE OF THE NEOTROPHIL ACTIVATING PROTEIN OF HELICOBACTER PYLORI (HP-NAP) AND/OR OF ITS PORTIONS AS ADJUVANTS FOR THE DEVELOPMENT OF T HELPER TYPE 1 (TH1) IMMUNE RESPONSES
<130> BE290F
<160> 7
<170> PatentIn version 3.2
<210> 1
   <211> 144
   <212> PRT
   <213> Helicobacter pylori
<220>
   <221> MISC_FEATURE
   <222> (46)..(101)
<220>
   <221> misc_feature
   <222> (46)..(46)
   <223> Xaa can be any naturally occurring amino acid
<220>
   <221> misc_feature
   <222> (73)..(73)
   <223> Xaa can be any naturally occurring amino acid
<220>
   <221> misc_feature
   <222> (101)..(101)
   <223> Xaa can be any naturally occurring amino acid
<400> 1
   Met Lys Thr Phe Glu Ile Leu Lys His Leu Gln Ala Asp Ala Ile Val
   1               5                   10                  15
   Leu Phe Met Lys Val His Asn Phe His Trp Asn Val Lys Gly Thr Asp
               20                  25                  30
   Phe Phe Asn Val His Lys Ala Thr Glu Glu Ile Tyr Glu Xaa Phe Ala
           35                  40                  45
   Asp Met Phe Asp Asp Leu Ala Glu Arg Ile Val Gln Leu Gly His His
       50                  55                  60
   Pro Leu Val Thr Leu Ser Glu Ala Xaa Lys Leu Thr Arg Val Lys Glu
   65                  70                  75                  80
   Glu Thr Lys Thr Ser Phe His Ser Lys Asp Ile Phe Lys Glu Ile Leu
                   85                  90                  95
   Glu Asp Tyr Lys Xaa Leu Glu Lys Glu Phe Lys Glu Leu Ser Asn Thr
               100                 105                 110
   Ala Glu Lys Glu Gly Asp Lys Val Thr Val Thr Tyr Ala Asp Asp Gln
           115                 120                 125
   Leu Ala Lys Leu Gln Lys Ser Ile Trp Met Leu Gln Ala His Leu Ala
       130                 135                 140
<210> 2
   <211> 36
   <212> PRT
   <213> helicobacter pylori
<400> 2
   Glu Ile Leu Lys His Leu Gln Ala Asp Ala Ile Val Leu Phe Met Lys
   1               5                   10                  15
   Val His Asn Phe His Trp Asn Val Lys Gly Thr Asp Phe Phe Asn Val
               20                  25                  30
   His Lys Ala Thr
           35
<210> 3
   <211> 34
   <212> PRT
   <213> helicobacter pylori
<400> 3
   Asn Thr Ala Glu Lys Glu Gly Asp Lys Val Thr Val Thr Tyr Ala Asp
   1 5 10 15
   Asp Gln Leu Ala Lys Leu Gln Lys Ser Ile Trp Met Leu Gln Ala His
   20 25 30
   Leu Ala
<210> 4
   <211> 36
   <212> PRT
   <213> helicobacter pylori
<400> 4
   Ala Thr Glu Glu Ile Tyr Glu Glu Phe Ala Asp Met Phe Asp Asp Leu
   1 5 10 15
   Ala Glu Arg Ile Val Gln Leu Gly His His Pro Leu Val Thr Leu Ser
   20 25 30
   Glu Ala Leu Lys
   35
<210> 5
   <211> 36
   <212> PRT
   <213> helicobacter pylori
<400> 5
   Leu Thr Arg Val Lys Glu Glu Thr Lys Thr Ser Phe His Ser Lys Asp
   1 5 10 15
   Ile Phe Lys Glu Ile Leu Glu Asp Tyr Lys His Leu Glu Lys Glu Phe
   20 25 30
   Lys Glu Leu Ser
   35
<210> 6
   <211> 247
   <212> PRT
   <213> Artificial
<220>
   <223> HP-NAP hybridized with CFP10 antigen of M. tuberculosis
<220>
   <221> MISC_FEATURE
   <222> (1)..(247)
<220>
   <221> misc_feature
   <222> (46)..(46)
   <223> Xaa can be any naturally occurring amino acid
<220>
   <221> misc_feature
   <222> (73)..(73)
   <223> Xaa can be any naturally occurring amino acid
<220>
   <221> misc_feature
   <222> (101)..(101)
   <223> Xaa can be any naturally occurring amino acid
<220>
   <221> misc_feature
   <222> (145)..(147)
   <223> Xaa can be any naturally occurring amino acid
<400> 6
   Met Lys Thr Phe Glu Ile Leu Lys His Leu Gln Ala Asp Ala Ile Val
   1               5                   10                  15
   Leu Phe Met Lys Val His Asn Phe His Trp Asn Val Lys Gly Thr Asp
               20                  25                  30
   Phe Phe Asn Val His Lys Ala Thr Glu Glu Ile Tyr Glu Xaa Phe Ala
           35                  40                  45
   Asp Met Phe Asp Asp Leu Ala Glu Arg Ile Val Gln Leu Gly His His
       50                  55                  60
   Pro Leu Val Thr Leu Ser Glu Ala Xaa Lys Leu Thr Arg Val Lys Glu
   65                  70                  75                  80
   Glu Thr Lys Thr Ser Phe His Ser Lys Asp Ile Phe Lys Glu Ile Leu
                   85                  90                  95
   Glu Asp Tyr Lys Xaa Leu Glu Lys Glu Phe Lys Glu Leu Ser Asn Thr
               100                 105                 110
   Ala Glu Lys Glu Gly Asp Lys Val Thr Val Thr Tyr Ala Asp Asp Gln
           115                 120                 125
   Leu Ala Lys Leu Gln Lys Ser Ile Trp Met Leu Gln Ala His Leu Ala
       130                 135                 140
   Xaa Xaa Xaa Met Ala Glu Met Lys Thr Asp Ala Ala Thr Gln Glu Ala
   145                 150                 155                 160
   Gly Asn Phe Glu Arg Ile Ser Gly Asp Leu Lys Thr Gln Ile Asp Gln
                   165                 170                 175
   Val Glu Ser Thr Ala Gly Ser Leu Gln Gly Gln Trp Arg Gly Ala Ala
               180                 185                 190
   Gly Thr Ala Ala Gln Ala Ala Val Val Arg Phe Gln Leu Ala Glu Ala
           195                 200                 205
   Ala Asn Lys Gln Lys Gln Glu Leu Asp Glu Ile Ser Thr Asn Ile Arg
       210                 215                 220
   Gln Ala Gly Val Gln Tyr Ser Arg Ala Asp Glu Glu Gln Gln Gln Ala
   225                 230                 235                 240
   Leu Ser Ser Gln Met Gly Phe
                   245
<210> 7
   <211> 242
   <212> PRT
   <213> Artificial
<220>
   <223> HP-NAP hybridized with ESAT-6 antigen of M. tuberculosis
<220>
   <221> misc_feature
   <222> (46)..(46)
   <223> Xaa can be amino acid Glu or Gly
<220>
   <221> misc_feature
   <222> (73)..(73)
   <223> Xaa can be amino acid Ile or Leu
<220>
   <221> misc_feature
   <222> (101)..(101)
   <223> Xaa can be amino acid Tyr or His
<220>
   <221> misc_feature
   <222> (145)..(147)
   <223> Xaa can be any naturally occurring amino acid
<400> 7
   Met Lys Thr Phe Glu Ile Leu Lys His Leu Gln Ala Asp Ala Ile Val
   1               5                   10                  15
   Leu Phe Met Lys Val His Asn Phe His Trp Asn Val Lys Gly Thr Asp
               20                  25                  30
   Phe Phe Asn Val His Lys Ala Thr Glu Glu Ile Tyr Glu Xaa Phe Ala
           35                  40                  45
   Asp Met Phe Asp Asp Leu Ala Glu Arg Ile Val Gln Leu Gly His His
       50                  55                  60
   Pro Leu Val Thr Leu Ser Glu Ala Xaa Lys Leu Thr Arg Val Lys Glu
   65                  70                  75                  80
   Glu Thr Lys Thr Ser Phe His Ser Lys Asp Ile Phe Lys Glu Ile Leu
                   85                  90                  95
   Glu Asp Tyr Lys Xaa Leu Glu Lys Glu Phe Lys Glu Leu Ser Asn Thr
               100                 105                 110
   Ala Glu Lys Glu Gly Asp Lys Val Thr Val Thr Tyr Ala Asp Asp Gln
           115                 120                 125
   Leu Ala Lys Leu Gln Lys Ser Ile Trp Met Leu Gln Ala His Leu Ala
       130                 135                 140
   Xaa Xaa Xaa Met Thr Glu Gln Gln Trp Asn Phe Ala Gly Ile Glu Ala
   145                 150                 155                 160
   Ala Ala Ser Ala Ile Gln Gly Asn Val Thr Ser Ile His Ser Leu Leu
                   165                 170                 175
   Asp Glu Gly Lys Gln Ser Leu Thr Lys Leu Ala Ala Ala Trp Gly Gly
               180                 185                 190
   Ser Gly Ser Glu Ala Tyr Gln Gly Val Gln Gln Lys Trp Asp Ala Thr
           195                 200                 205
   Ala Thr Glu Leu Asn Asn Ala Leu Gln Asn Leu Ala Arg Thr Ile Ser
       210                 215                 220
   Glu Ala Gly Gln Ala Met Ala Ser Thr Glu Gly Asn Val Thr Gly Met
   225                 230                 235                 240
   Phe Ala

## Description

### Field of the invention

The present invention relates to the use of the Neutrophil Activating Protein of *Helicobacter pylori* (HP-NAP) and/or of its portions as adjuvants for the development of T helper type 1 (Th1) immune responses.

### Background art

As is known, human CD4 T lymphocytes can be divided into at least three distinct functional subsets based on their cytokine secretion profile: CD4⁺ T-cells able to produce interferon (IFN)-γ (and IL-2), but not interleukin (IL)-4 or IL-5, are categorized as T helper type 1 or Th1; T cells able to produce IL-4 and IL-5, but not IFN-γ, are coded as T helper type 2 or Th2; and T cells producing both IFN-γ and IL-4/IL-5 are coded as Th0 [Del Prete, G. et al., J. Clin. Invest. 88, 346-350, (1991)]. A similar functional distinction between type 1, 2 and 0 (Tc1, Tc2 and Tc0) has been proposed also for the human cytotoxic CD8 T cells. Human Th1 and Th2 cells differ not only for their profile of interleukin or cytokine production, but also for cytolytic potential. The great majority of Th1, but only few Th2, cells exhibit lectin-dependent and MHC-restricted cytotoxic activity. The majority of Th0 cells also exhibit both lectin-dependent and MHC-restricted cell cytotoxicity, but their efficiency in killing cell targets is usually lower than that of Th1 cells [Del Prete, G., et al., J. Exp. Med. 174, 809-813, (1991)].

After challenge with microbial or inflammatory stimuli, immature DCs undergo maturation and upregulation of MHC and costimulatory molecules that are essential for T cell priming, and migrate to lymphoid organs. DCs are a major source of IL-12, a cytokine that is key in innate immunity and drives Th1 polarization of T cell responses. Like IL-12, IFN-α (and IFN-γ) promotes the differentiation of CD4 T cells into Th1, both in vitro and in vivo. On the other hand, in vitro and in vivo evidence demonstrates that IL-4 has reciprocal activity, inducing T-cell differentiation into Th2 cells. The ability of Th1 cells in mediating killing of antigen-presenting cells (APC) is responsible for the down-regulation of specific B cell responses, accounting for short-lived antibody production frequently observed in successful inflammatory reactions associated with vigorous Th1-mediated delayed-type hypersensitivity reactions. By contrast, repeated and chronic stimulation by 20-40 kDa protein antigens, such as inhaled allergens or helmith parasite antigens, does not result in strong macrophage/DC activation and therefore does not evoke high production of IL-12 and other Th1-polarizing cytokines, but allows the activation of Th2 cells that produce IL-4. The inability of allergen-specific Th2 cells to kill their APC can explain, at least in part, the chronicity of IgE antibody production occurring in atopic patients.

DCs, the major antigen-presenting cells (APC), play an important role in the immune response against infections. Their function is crucial in the regulation of both the early innate immune responses and the subsequent adaptative immunity [Bancherau, J. et al., Nature 392: 245-252 (1998)]. The process of differentiation from an immature DC into a mature professional APC can be induced by whole bacteria or their components, the so-called pathogen-associate molecular patterns (PAMPs). This process is accompanied by up-regulation of major histocompatibility complex (MHC) classe II and costimulatory molecules, together with cytokine production [Reis e Sousa, C., et al., Curr. Opin. Immunol. 11, 392-399, (1999.); Dixon, G.L., et al., Infect. Immun. 69, 4351-4357 (2001)].

The research by Kranzer K. et al. [Infect. Immun. 72, 4416-4423 (2004)], carried out with *Helicobacter pylori* and immature DCs, revealed that *H. pylori* induced immature DCs to release several cytokines (such as IL-6, IL-10 and IL-12) and also contributed to their maturation; however which was the bacterial factor responsible for such effects remained unknown. The research by Dixon et al. [Infect. Immun. 69, 4351-4357 (2001)], carried out on group B *Neisseria meningitidis*, also showed pathogen-induced dendritic cell activation and cytokine production, including IL-12, in the host.

A general feature of the host response to the *H. pylori* infection is an infiltration of the sub-epithelial gastric lamina propria by phagocytes, mainly monocyte/macrophages in addition to neutrophil granulocytes, and lymphocytes [Dixon, M.F., et al., Am. J. Surg. Pathol. 20, 1161-1181 (1996)]. The two major *H. pylori* virulence factors described so far are the Vacuolating cytotoxin (VacA) and the Neutrophil Activating Protein (HP-NAP) [Reyrat, J.M. et al., Mol. Microbiol. 34, 197-204 (1999); Montecucco, C. & de Bernard, M., Microbes Infect. 5, 715-721 (2003)]. HP-NAP, an oligomeric protein of 150 kDa, was initially reported as a promoter of endothelial adhesion of neutrophils and was designated as Neutrophil Activating Protein because it stimulates high production of oxygen radicals from neutrophils [Evans, D.J. Jr. et al., Infect. Immun. 63, 2213-2220 (1995)]. In addition, HP-NAP increases in monocytes the synthesis of tissue factor and the secretion of type-2 plasminogen activator inhibitor [Montecucco et al., cit.; Montemurro, P. et al., J. Infect. Dis. 183, 1055-1062 (2001)]. In addition, HP-NAP is chemotactic for neutrophils [Montecucco, C. *et al*., cit.].

Different types of T cell responses and cytokine secretion patterns are elicited by infections with pathogens and during the subsequent inflammatory processes, which are crucial for infection healing and protection or for pathogen persistence and pathology. On the other hand, in some immunopathological conditions, fully polarized T helper cell reponses are responsible for disease. Allergy, in which polarized Th2 responses, abundant IL-4 secretion and IgE antibody response are inappropriate and harmfull, is just an example [Del Prete, G., et al., Eur. J. Immunol. 23, 1445-1449, 1993]. Other Th2-mediated conditions include responses to parasitic infestations, other chronic infections and some aspects of systemic autoimmune diseases.

The current treatment of allergic diseases is based on pharmaco-therapy and allergen immunotherapy (AI). Immunotherapy, where appropriate, should be used in combination with the other forms of therapy with the goal that the allergic patient will become as symptom-free as clinically possible.

Allergen immunotherapy (AI) should inhibit both early and late responses to allergen exposure. In general, AI is accompanied by increases in allergen-specific IgG, particularly of the IgG4 isotype, which inhibits not only IgE-dependent histamine release from mast cells, but also IgE-mediated allergen presentation to T cells. AI is supposed to act on T cells possibly changing peripheral and mucosal Th2 responses to allergen in favor of Th1 responses.

Novel approaches to AI currently being explored include the use of adjuvants, such as monophosphoryl lipid A or nucleotide immunostimulatory sequences derived from bacteria that potentiate Th1 responses. Alternative strategies include the use of allergen-derived peptides or modified recombinant allergen vaccines that act on T cells while minimizing the IgE-dependent mast cell activation that is dependent on the native allergen conformation [Till, S.J. et al. J. Allergy Clin. Immunol. 113:1025-34, 2004].

It appears that in the case of chronic infections with pathogens, the balance between Th1 and Th2 immune responses becomes gradually upset. As disease progression occurs, there is a decrease in the cell-mediated response with a Th1 to Th0/Th2 shift, a phenomenon that can be clearly seen in HIV infection [Maggi, E. et al. Science 265: 244-248, 1994]. Taking into consideration the Th1 enhancing effects that IL-12 presents, there is an increasing interest in the potential manipulating the immune system in order to treat HIV and other chronic infections. Apparently, there is a direct correlation between the levels of IL-12 and cellular immunity as the activity of this cytokine stimulates the cell-mediated response. With regard to treatment with IL-12, researchers are encouraged that the weakened human immune response to the pathogen may be empowered and gradually decrease or stop the disease development. Yet aside from use in the management of cancer (see below),recombinant IL-12 is also being studied to examine its safety and efficacy in the treatment of HIV or other severe infections, especially when administered in conjunction with standard antiretroviral or appropriate antibiotic treatment.

Biological therapy (immunotherapy, biotherapy, or biological response modifier - BRM - therapy) is a promising new addition to the modern vaccine strategies and to the family of cancer treatments. Biological therapies use the body's immune system, either directly or indirectly, to fight cancer or to lessen side effects that may be caused by some cancer treatments. The immune system may recognize the difference between healthy cells and cancer cells in the body and eliminate those that become cancerous. Cancer may develop when the immune system breaks down or is overwhelmed. Biological therapies are designed to repair, stimulate, or enhance the immune system's natural anticancer function. IL-2 and IL-12 have been the most widely studied in cancer treatment. Tumor vaccines are another form of biological therapy currently under study. Researchers are developing tumor vaccines that encourage the immune system to recognize cancer cells. Tumor vaccines may help the body reject tumors and also help prevent cancer from recurring. Researchers are also investigating ways that tumor vaccines can be used in combination with BRMs. IL-12 is one candidate that held early promise. This proinflammatory and immunoregulatory cytokine plays a central role in the interaction between innate resistance and adaptive immunity by inducing IFN-γ production and generating Th1 responses and cytotoxic T lymphocytes. In several studies comparing different cytokines, IL-12 was the most effective in terms of tumor eradication, anti-metastatic activity, and eliciting long-term antitumor immunity. Clinical trials have however demonstrated that excessive toxicity limits the clinical application of exogenous IL-12. Therefore, local and efficient expression of endogenous IL-12 or harnessing the anticancer properties of other cytokines is being investigated as an alternative approach. Like other forms of cancer treatment, biological therapies can cause various side effects, which can vary widely from patient to patient and often be severe with need of close patient monitoring during treatment.

Compounds other than cytokines are known to boost the activity of the immune system and are now under study as possible adjuvants, particularly for use with vaccines. Some of the most commonly studied adjuvants are: a) *Bacille Calmette-Guérin (BCG)*: a bacterium that is related to *M. tuberculosis*. BCG does not cause serious disease in humans, but it infects human tissues, attracts immune system cells to areas of injection, and activates the immune system. It is FDA approved as a routine treatment for superficial bladder cancer. Its usefulness in other cancers as an adjuvant is also being tested. b) *Aluminum hydroxide (alum), aluminum phosphate, aluminum sulphate*: Alum is one of the most common adjuvants used in clinical trials for cancer vaccines. It is already used in vaccines against several germs, including the hepatitis B virus; c) oil-in-water emulsion formulations (with or without other specific immunostimulating agents such as muramyl peptides or bacterial cell wall components), such as MF59^{™}, SAF, the Ribi^{™} adjuvant system (RAS); d) saponin adjuvants, such as Stimulon^{™}; e) mucosal adjuvants, such as non toxic portions of bacterial toxins, LTK63 and others, have been specially designed for mucosal immunization protocols. Exogenous cytokines, including IL-1, IL-2, IL-4, IL-6, IL-7, IL-12, interferons (e.g. IFN-γ), macrophage colony-stimulating factor (M-CSF) and tumor necrosis factor (TNF-α) have been used to enhance the efficacy of the candidate vaccines. IL-12 apparently has the capability to stay active in the body for lengthier periods of time, which makes it an even more praiseworthy option in human clinical trials.

### Summary of the invention

The invention deals with the idea that HP-NAP, or a portion of that molecule, through the induction of cytokine secretion (namely IL-12, IL-23) by cells of innate immunity, exerts an adjuvant effect on T lymphocyte development, shifting to Th1 immune responses that would otherwise be of Th0 or Th2 type. In this regard, robust evidence has been obtained that HP-NAP exerts in vitro the same effect of exogenous IL-12 in inducing Th1 polarization of ongoing T-cell responses, regardless of the nature of the inducing antigen.

As mentioned above, the great majority of Th1, but only few Th2, cells exhibit cytotoxic activity. The majority of Th0 cells also exhibit cytotoxicity, but their efficiency in killing cell targets is usually lower than that of Th1 cells [Del Prete, G., et al., J. Exp. Med. 174, 809-813, (1991) ]. Our finding that HP-NAP is able to influence not only the cytokine profile of antigen- or allergen-specific T cells, but also enables them to kill cell targets is of great interest in view of new approaches to the therapy of Th2-mediated disorders.

HP-NAP induces the production of both IL-12 and IL-23. Recently, factors other than IL-12 able to drive Th1 differentiation have been discovered, such as IL-23, which shares the p40 chain with IL-12, but pairs this with a unique p19 chain. Showing the ability to induce both the maturation of monocytes into dendritic cells, and the up-regulation of IL-12 and IL-23 secretion by both neutrophil granulocytes and monocytes following the binding to the Toll-like Receptor (TLR)2, HP-NAP represents a possible optimal substitute of exogenous cytokines (e.g. IL-12 and IL-23) in strategies aimed to provide Th1-polarizing signals at the onset or in the course of a T cell response. In other words, HP-NAP, by favoring the deployment by DCs of their Th1-polarizing capacity, acts as a powerful Th1-polarizing signal and to re-direct the generation of Th0/Th2 responses into a more appropriate and non pathogenic class. Likewise, HP-NAP can find a therapeutic role in immunotherapy protocols with ex vivo-treated DCs and in the strategies of strong systemic IL-12 induction for optimizing allergen immunotherapy, the treatment of parasitic infections and other Th2-dominated conditions, for enhancing the Th1-mediated protective effects of vaccines against pathogens and for cancer immunotherapy.

The use of HP-NAP or of the polypeptide sequences expressing its active adjuvant domain(s) more closely mimics the physiologic activation of macrophage/DCs than exogenous cytokines can do.

A major advantage of HP-NAP is that DCs stimulated by it, progressively modulate the delivery of Th1-polarizing cytokines at physiological doses, avoiding thus the well known side effects of the direct in vivo administration of IL-12 and other Th1-polarizing cytokines. Moreover, the maturation effect of HP-NAP on macrophage/DCs in vitro, is extremely useful in the context of immunization protocols based on the *in vitro* priming of *ex vivo* obtained autologous DC before their reinfusion into the recipients. *In vitro* conditioning with HP-NAP/peptides of DCs at the time of the challenge with the target antigen(s), would induce DCs to fully deploy their Th1-polarizing capacity and their potential of re-directing the generation of the T cell responses into the Th1 functional profile.

Besides by the entire HP-NAP molecule, the Th1-inducing adiuvant effect of HP-NAP is mimiked also by some of its active domains. The possibility to obtain by chemical synthesis those active domains (20-40 amino acids) with biological activity, makes it feasible a protocol of induction of Th1 responses by using the entire HP-NAP protein, followed by the administration of one or more of the active polypeptides.

Such a protocol would have the advantage of minimizing the possible inhibitory effects mediated in the host by an anti-HP-NAP antibody response, that might interfere with the Th1 adjuvanticity of the entire protein.

Regardless of the acidic gastric environment, HP-NAP is active *in vivo* in the gut, where it elicits a predominant Th1 response to a number of *H. pylori* antigens in the mucosal lymphocytic infiltrates of *H. pylori-*induced chronic gastritis. Therefore, while the adjuvant activity of other molecules can be hampered by acid pH, it is reasonable to think that HP-NAP/peptide adjuvant effect is not lost at acid pH in the stomach, allowing its/their administration being easily delivered by either parentral and mucosal or transcutaneous route, but also efficiently by oral route. The possibility of delivering HP-NAP by oral route without loss of its activity allows the feasibility of Th1-conditioning protocols in which crude preparations of HP-NAP are used, like those obtained from edible plants (e.g. potato or tobaco plant) transfected with the HP-NAP gene. This type of HP-NAP preparations would have the advantage of getting massive preparations of huge amounts of HP-NAP that can be easily standardized for their potency with very low costs in comparison with other technologies of recombinant protein synthesis.

Taking into consideration the concepts reported above, it emerges that the main object of the present invention is Neutrophil Activating Protein from *Helicobacter pylori* (HP-NAP) or immmunologically equivalent fragments thereof for enhancing in humans or animals the Th1-type polarization of, or for redirecting spontaneous and undesired Th0/Th2 type into Th1-type, T-cell protective immune responses induced by allergens, endogenous or exogenous antigens, other than antigens of *Helicobacter pylori*, as reported in the claims 1 to 9 and 11 to 14.

A second object of the invention, is HP-NAP or immmunologically equivalent fragments thereof for use in an *ex vivo* method for the induction of macrophage/DCs maturation *in vitro*, the enhancement of their antigen-presenting cell (APC) function, and deployment of their Th1-polarizing capacity before their re-infusion into the original donors with the aim of eliciting effective Th1-mediated responses to antigen(s) of pathogens or tumors, including the phase in which macrophage/DCs are treated *in vitro* with NH-NAP protein from *Helicobacter pylori* or with its immunologically equivalent fragments, as reported in claim 10.

A further object of the invention is a vaccine composition comprising (a) one or more antigens with the exception of antigens of *Helicobacter pylori,* (b) one or more pharmaceutically acceptable excipients and optional carriers or other immunoregulatory agents, (c) and, as an immune adjuvant, a therapeutically effective amount of the NAP protein of *Helicobacter pylori* (HP-NAP), or immunologically equivalent fragments thereof for use in a treatment against pathogens, with the exception of treatment of *Helicobacter pylori* infections, as reported in claims 15 to 23.

A further object of the invention is a method of preparation of the composition according to any one of claims from 15 to 23, **characterized in that** therapeutically effective amounts of HP-NAP from *Helicobacter pylori* or immmunologically equivalent fragments thereof are mixed with one or more antigens or allergens and with excipients and additives usually appropriate for a vaccine composition, as reported in claims 24 and 25.

Futher objects will be made clear by the detailed description reported below.

### Brief description of the drawings

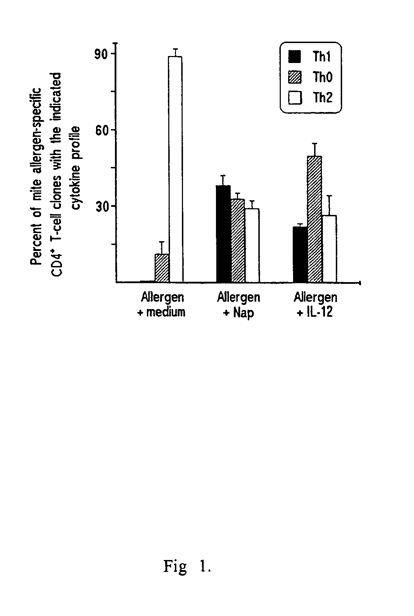

**Figure 1** shows the cytokine profile of allergen-specific CD4⁺ clones generated from allergen-induced T-cell lines conditioned with medium alone, HP-NAP or IL-12. Allergen-induced T-cell lines were generated from peripheral blood mononuclear cells (PBMC) of house dust mite allergen-sensitive donors and medium, HP-NAP or IL-12 were added at the time of allergen exposure *in vitro*. T-cell blasts of each line were then cloned and allergen-specific T cell clones were stimulated for 48 hours with medium or mite allergen in the presence of irradiated autologous APC. Culture supernatants were then collected and assayed for their IFN-γ and IL-4 content. Clones able to produce IFN-γ, but not IL-4, were categorized as Th1, clones producing IL-4 but not IFN-γ were coded as Th2, whereas clones producing both IFN-γ and IL-4 were categorized as Th0. Results represent mean percent proportions (±SD) of clones with the indicated cytokine profile.

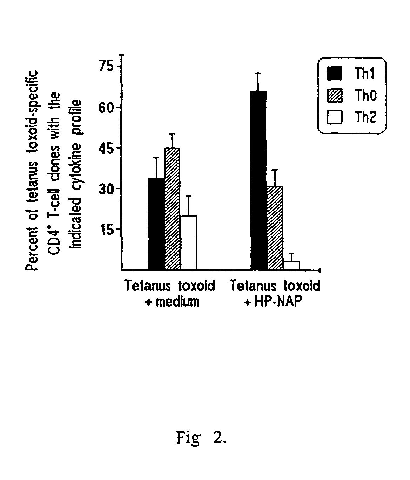

**Figure 2** shows the shifting to Th1 of the cytokine profile of tetanus toxoid-specific CD4⁺ clones generated from tetanus toxoid-induced T-cell lines conditioned with HP-NAP. Tetanus toxoid-induced T-cell lines were generated from PBMC of normal donors and medium or HP-NAP were added at the time of antigen stimulation *in vitro*. T-cell blasts of each line were then cloned and tetanus toxoid-specific T cell clones were stimulated for 48 hours with medium or tetanus toxoid in the presence of irradiated autologous APC. Culture supernatants were then collected and assayed for their IFN-γ and IL-4 content. Results represent mean percent proportions (±SD) of clones with the indicated cytokine profile.

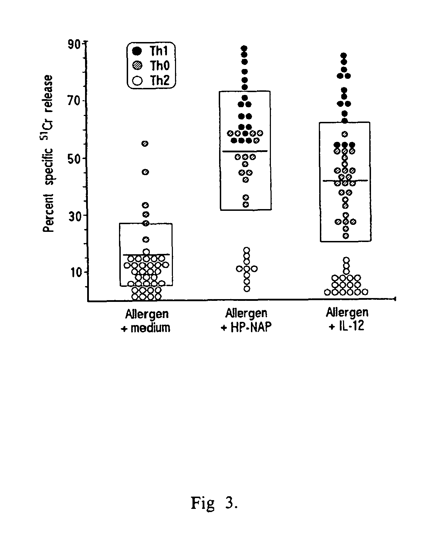

**Figure 3** shows the cytotoxic activity of Th clones derived from allergen-induced T-cell lines conditioned with medium alone, HP-NAP or IL-12. Results represent the percent specific ⁵¹Cr-release induced by single clones in PHA-treated murine ⁵¹Cr-labeled P815 cells at an effector to target ratio of 5 to 1. Orizontal bars and boxes indicate means values ±SD, respectively.

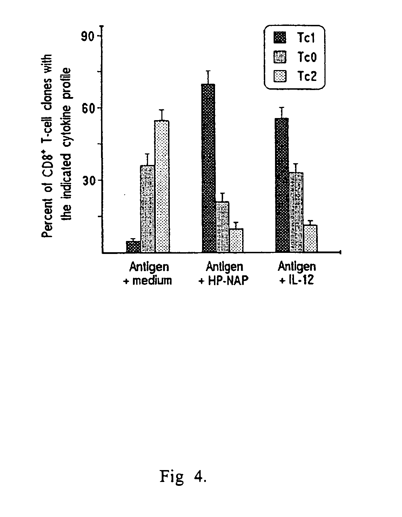

**Figure 4** shows the shifting from Tc2/Tc0 to Tc1 of the cytokine profile of CD8⁺ clones with unknown specificity generated from allergen-induced T-cell lines conditioned with HP-NAP. CD8⁺ T-cell clones with undefined specificity and unable to proliferate upon re-challenge with mite allergen were assessed for their cytokine profile and cytotoxic activity. CD8⁺ clones with high cytotoxic activity and able to produce IFN-γ, but not IL-4, were categorized as Tc1, CD8⁺ clones lacking cytotoxic activity and producing IL-4, but not IFN-γ were coded as Tc2, whereas CD8⁺ clones with intermediated cytotoxic activity and producing both IFN-γ and IL-4 were categorized as Tc0. Results represent mean percent proportions (±SD) of clones with the indicated functional profile.

### Detailed description of the invention

### Methods of preparation and purification of HP-NAP.

HP-NAP production/purification is performed from *Bacillus subtilis* transformed with a HP-NAP encoding plasmid. Briefly, from a 12-hours liquid culture in LB medium (NaCl 10 g/l, bacto-teryptone 10 g/l, yeast extract 2.5 g/l), plus chloramphenicol 20 µg/ml, bacteria suspension are diluted 50 times in the same medium and incubated for 8 h at 37°C. After a dilution 1:100 in YT medium (NaCl 5 g/l, bacto-teryptone 16 g/l, yeast extract 10 g/l) plus chloramphenicol 20 □g/ml, bacteria are left to grow for 15 h at 37°C, before being collected by centrifugation for 10 min at 6000 g at 4°C. Pellet is resuspended in PBS LPS-free plus proteases inhibitors and the suspension is lysed through the French Press apparatus at 160 atm. After centrifugation at 32000 g for 30 min at 4°C to eliminate membrane debris, proteins are precipitated with (NH₄)₂SO₄ 70% w/v. Soluble fraction, collected by centrifugation as above, is dyalized in Na₂HPO₄ 30 mM, NaCl 0.5 M, pH 7.8, concentrated and filtered. On the basis of its 9 Histidines, HP-NAP is purified from the protein solution with an affinity column loaded with Ni²⁺ in a FPLC apparatus. Elution is carried out with an hymidazole gradient from 0 to 100 mM in Na₂HPO₄ 30 mM, NaCl 0.5 M, pH 7.8 as it follows: from 0 to 40 mM in 30 ml, from 40 to 100 mM in 10 ml and then at 100 mM for 20 ml with a flow rate of 1 ml/min. Fractions containig HP-NAP are dyalized in PBS LPS-free before freezing in liquid nitrogen and storing at -80°C.

The protein purity is judged from overloaded gels composed of different percentages of polyacrylamide, run according to Laemmli (1970). Mass spectrometry analysis confirmes that the protein consisted of a single molecule of 16 875 ± 20 Da. Protein concentration is determined by absorbance at 280 nm using a spectrophotometer.

### Analytical and control methods

Regardles of the methods of synthesis, each preparation of HP-NAP or HP-NAP analogue or peptide(s) or HP-NAP-antigen hybrid(s) is tested for:
a) degree of potency of induction of IL-12 and/or IL-23 expression in human neutrophils and/or monocytes,
b) degree of potency of shifting human Th2-type responses into Th1,
c) degree of potency of shifting non cytolytic Th2 into cytolytic Th1 T-cell responses,
d) lack of side effects in cultures of human cells, and in experimental animals before testing in human volunteers.

The reported basic HP-NAP amino acid sequence reported in the literature is as follows, with three possible amino acid substitutions that generate eight possible combinations:

### Definition of the dominant T-cell epitopes of HP-NAP recognized by HP-NAP-specific T cells derived from the gastric infiltrates induced by H. pylori.

In order to define the T-cell epitopes of HP-NAP recognized by the CD4 HP-NAP-specific gastric T cell clones, the proliferative response of each clone was assessed in the presence of irradiated autologous APC and a series of 29 overlapping (by 5 amino acids on both sides) peptides of 15 amino acids each, globally encompassing the entire amino acid sequence of HP-NAP. Interestingly, 48% of the HP-NAP-specific gastric T cell clones from different donors recognized epitopes clustered at the N-terminal within the amino acid sequence 5-40 (SEQ ID NO:2) EILKHLQADAIVLFMKVHNFHWNVKGTDFFNVHKAT, and that and 20% of clones recognized their epitope at the opposite terminal portion, 110-144 residues of HP-NAP (SEQ ID NO:3) NTAEKEGDKVTVTYADDQLAKLQKSIWMLQAHLA, whereas the other few clones reacted to epitopes scattered in other portions of the protein, such as the amino acid sequences 39-74, (SEQ ID NO:4) ATEEIYEEFADMFDDLAERIVQLGHHPLVTLSEALK, and 75-110, (SEQ ID NO:5) LTRVKEETKTSFHSKDIFKEILEDYKHLEKEFKELS. These data suggest that the most immunologically active portions of HP-NAP are located in either the N-terminus or the C-terminus of the protein.

### Molecular hybrids including HP-NAP and vaccine antigen(s) or allergen(s)

Appropriate combinations of gene sequences encoding different molecules result in molecular fusion hybrids that express multiple epitopes and may show better immunogenicity than the corresponding single components including either antigens or antigens and protein adjuvants in hosts undergoing vaccines bases on those antigens.

In the case of the relatively small HP-NAP protein, a feasible method of coupling both immunogenicity and adjuvanticity in a vaccine strategy is to get hybrid proteins coded by hybrid genes encoding one or more of the immunologically relevant antigen(s) or allergen(s) and the HP-NAP gene or the portion of the HP-NAP gene encoding the adjuvant active domains. Examples of such hybrids are represented by fusion proteins with the sequence reported below which include HP-NAP protein hybridized with a vaccine antigen selected between CFP10 or ESAT-6 of *M. tuberculosis* already taken into consideration as vaccine candidates for the prevention of tuberculosis. in which XXX broadly represent a conjugation oligopeptide including 1, 2, 3, 4, or more amino acid residues.

The expression and purification systems of the hybrids would be the same of those used for HP-NAP, with possible minor modifications in case of hybrids containing only one or more domains of HP-NAP.

### Applications

The present invention is based on the discovery that HP-NAP exerts an adjuvant activity for the development of preferential human Th1 responses to antigens. In this document, HP-NAP or HP-NAP analogue proteins or their peptides are referred to as "the adjuvant", whereas the combination of antigen(s) with HP-NAP or HP-NAP analogue proteins or their peptides are referred to as the "adjuvanted immunogens".

According to the present invention there is provided a method for:
- enhancing the Th1 polarization of T-cell responses in humans undergoing vaccines with one or more antigens, regardless of the type of antigen(s), its(their) molecular configuration or the immunization route, with the single exception that such antigen(s) are antigen(s) from *Helicobacter pylori* and/or aimed to the treatment of *Helicobacter pylori* infections,
- redirecting to Th1 undesired spontaneous T-cell responses to allergens, antigens or autoantigens,
- redirecting to Tc1 undesired spontaneous CD8 Tc0/Tc2 T-cell responses to exogenous or endogenous antigens,
- enhancing low efficiency or poor Th1-type responses induced by conventional immunization protocols or poorly effective spontaneous responses, such as those to cancer antigens,
- induction of endogenous IL-12 and IL-23 production,
- induction *in vitro* and/or *in vivo* of dendritic cell maturation and enhancement of their antigen-presenting cell (APC) function.

Preferably, administration route is mucosal, including conjunctivae, upper or lower airways or gut. For specific applications, administration can be cutaneous or parenteral, more preferably, intramuscular.

The one or more antigen(s) or allergen(s) administered as adjuvanted immunogens are all those able to elicit specific immune responses in animals, preferably mammals, more preferably humans.

The adjuvant and the adjuvanted immunogens are obtained by various usual methods, i.e. by purification/isolation from cell culture, recombinant technology or by chemical synthesis.

The adjuvant and the adjuvanted immunogens may be in association with one or more pharmaceutically acceptable excipients.

The adjuvanted immunogens of the pharmaceutical composition can include antigens of all the pathogens (except *Helicobacter pylori*) or allergens against which the induction of a substantial Th1-type response in the host is desired.

The pharmaceutical composition of the invention can be an immunogenic composition such as, but not necessarily limited to, a vaccine.

The adjuvant and the adjuvanted immunogens based on HP-NAP according to the invention may either be prophylactic (i.e. to prevent infection and/or disease status) or therapeutic (i.e. to treat diseases regardless of their pathogenesis).

The compositions of adjuvant and the adjuvanted immunogens (e.g. the antigen, a pharmaceutically acceptable carrier, and the adjuvant) typically will contain diluents, such as water, saline, glycerol, ethanol, etc. Additionally, as auxiliary substances, such as wetting or emulsifying agents, pH buffering substances, and the like, may be present among excipients.

Typically, the adjuvant and the adjuvanted immunogens are prepared as injectables, either as liquid solutions or suspensions, regardless of the administration route; solid forms suitable for solution in, or suspension in, liquid vehicles prior to administration may also be prepared. The preparations also may be emulsified or encapsulated in liposomes for special needs.

Edible transgenic plants for the production and delivery of the adjuvant and the adjuvanted immunogens may also be used.

It is also possible the use of transgenic edible plants for the production and administration of the adjuvant and the adjuvanted immunogens.

Adjuvanted immunogenic compositions used as vaccines comprise an immunologically effective amount of the antigenic polypeptides, as well as any other of the HP-NAP-related components, in appropriate amounts. By "immunologically effective amount", it is meant that the administration of that amount to an individual, either in a single dose or as part of a series of doses, is effective on the basis of the therapeutic or preventive aims. This amount varies depending on the nature of the antigen(s), the health and general conditions of the individuals to be treated, the group of individual to be treated (e.g. non-human primate, primate, etc.), the capacity of the individual's immune system to set up cell-mediated and/or humoral responses, the degree of protection desired, the formulation of the vaccine, the treating doctor's assessment of the medical situation, and other relevant factors. It is expected that the amount will fall in a relatively broad range that can be determined through routine clinical trials.

The immunogenic compositions are administered mucosally or non-mucosally, more specifically parenterally i.e. by injection, either subcutaneously or intramuscularly. Transdermal or transcutaneous administration may also be used. Dosage treatment may be concentrated into a single dose schedule or fractionated in a multiple dose schedule. The adjuvant and the adjuvanted immunogen may be administered in conjuction with other immunoregulatory agents.

### Experimental results

The invention is based on the feasibility of shifting preferential Th0/Th2 type human T cell responses into preferential Th1 responses by the use of *H*. *pylori* Neutrophil Activating Protein (HP-NAP). It has been shown that, through its binding to Toll-like Receptor (TLR)-2, HP-NAP stimulates either human neutrophils or monocytes to remarkably increase their expression of IL-12, a key cytokine able to promote the differentiation of naive T helper (Th) cells into cells with the Th1 phenotype. HP-NAP also induces monocytes to produce IL-23 and to differentiate into mature dendritic cells. These results indicate that HP-NAP has the potential of triggering a T hepler type 1 differentiation program in T cells undergoing specific antigen challenge. In fact, addition in culture of HP-NAP to allergen-induced T-cell lines generated from mite allergen-sensitive donors resulted in a shift from a predominant Th2 to a Th1 phenotype of allergen-specific T cells. An *in vivo* correlate of the Th1-polarizing capacity of HP-NAP was provided by the consistent Th1-polarization of the functional profile of a series of HP-NAP-specific CD4 T-cell clones generated from in vivo activated T cells derived from the gastric mucosa of *H*. *pylori* infected patients with peptic ulcer. Taken together, these data indicate that HP-NAP, besides being a major *H*. *pylori* antigen recognized by T cells at gastric level, also represents a powerful adjuvant for the induction of Th1 responses in humans.

### 1.1. HP-NAP stimulates IL-12 and IL-23 production in neutrophil granulocytes

Total RNA was extracted from neutrophils incubated with HP-NAP, retrotranscribed and subsequently amplified by Real Time PCR in the presence of appropriated primers. A significant TNF-α mRNA expression was found after 6 hours of incubation before dropping down after 8 hours. More importantly, the amount of mRNA for IL-12p35 and for IL-12p40 were also remarkably increased, though with different kinetics and degree: IL-12p40 mRNA increased more than IL-12p35 messenger. While the expression of IL-12p40 peaked at 6 hours, the increased expression of p35 already observed at 6, reached its maximum value at 12 hours. For both messengers no further modulation was detectable at 24 hours. The kinetics of cytokine mRNA levels determined by quantitative RT-PCR were consistent with the kinetic of IL-12 protein accumulation in the culture supernatant. The p40 subunit associates not only with IL-12p35, but also with another molecule, p19, to form IL-23. In human neutrophils, treatment with HP-NAP resulted in a prompt up-regulation of IL-23p19 mRNA that decreased thereafter.

### 1.2. HP-NAP stimulates IL-12 and IL-23 production in monocytes

HP-NAP was extremely efficient in up-regulating the monocyte expression of the IL-12p35, IL-12p40 and IL-23p19 mRNAs. In particular, IL-12p35 mRNA showed its peak at 30 minutes, whereas IL-12p40 mRNA peaked between 8 and 12 hours, and was still detectable at 24 hours. The kinetics of p40 and p35 IL-12 mRNA levels were consistent with the kinetic of p70 IL-12 protein accumulation in the culture supernatant. Following HP-NAP stimulation, the monocyte expression of IL-23p19 mRNA peaked at 2 hours, followed by a progressive decrease. Taken together these findings indicate the HP-NAP acting either on neutrophils or on monocytes, creates a cytokine milieu enriched in IL-12 and IL-23, which drive the differentiation of antigen-stimulated T cells toward a polarized Th1 phenotype.

### 1.3. HP-NAP stimulates monocytes to differentiate into mature dendritic cells

While control monocytes incubated with medium progressively died between 28 and 36 hours, monocytes incubated with HP-NAP survived longer. In addition, HP-NAP-treated monocytes revealed a progressive shape modification and a tendency to cluster and to detach from the substrate. Compared to the corresponding baseline values, the expression of HLA-DR markedly increased at day 5 of incubation with HP-NAP (about 10 folds) and even more (about 20 folds) at day 7. CD80 expression as well significantly increased (about 20 folds) at day 5 in comparison to its baseline expression and even more (about 40 folds) at day 7. Likewise, the expression of CD86, already increased after 5 days, was remarkably higher (about 15 folds) at day 7. No significant change of the expression of the markers was observed in untreated control cells. These results suggest that HP-NAP induces the differentiation of monocytes into mature dendritic cells.

### 1.4. HP-NAP activates cells of innate immunity through the interaction with TLR-2

The ability of HP-NAP to bind Toll-like Receptors (TLR) was investigated by recombinant HEK-293 cell line that functionally expresses a given TLR protein. HP-NAP activated specifically human TLR-2 in the system at the same way and level of the positive control (PAM2), whereas no significant activity/binding was found for HEK-293 cell lines expressing TLR-3, 4, 5, 6, 7, 8, or 9.

### 1.5. HP-NAP shifts the development of allergen-specific T cells from Th2 to Th1 phenotype

In order to assess whether HP-NAP had any effect on the *in vitro* development of Th1/Th2 type responses, allergen-induced T-cell lines were generated from peripheral blood mononuclear cells (PBMC) of house dust mite allergen-sensitive donors and medium, HP-NAP or IL-12 were added. Stimulation with mite allergen resulted in the expansion of T-cell lines. T-cell blasts of each line were recovered and cloned by limiting dilution according to a high efficiency protocol. Of the 38 CD4⁺ clones generated from the allergen-induced lines conditioned with medium, 18 (47%) were reactive to mite allergen, whereas the other 20 CD4⁺ clones of this series failed to significantly proliferate in response to mite allergen. In the series of clones generated from T-cell lines conditioned with HP-NAP, 21 (52.5%) out of the 40 CD4⁺ clones proliferated upon allergen challenge, whereas in the series of clones generated from T-cell lines conditioned with IL-12, the allergen-specific CD4⁺ clones were 42%. In the series of allergen-specific clones from the allergen-induced T cell lines conditioned with medium, none of the 18 clones expressed a Th1 profile, 11% were Th0 producing both IFN-γ and IL-4, whereas 89% were Th2 clones (Figure 1). In contrast, in the series of allergen-specific T-cell clones from the allergen-induced lines conditioned with HP-NAP, as many as 38% were Th1, 33% were Th0, whereas Th2 clones were only 29%. As expected, conditioning with IL-12 of the allergen-induced lines resulted in the development of a series of allergen-specific clones including 22% Th1, 52% Th0 and 26% Th2 (Figure 1).

In conclusion, like that of IL-12, addition in culture of HP-NAP at the time of allergen stimulation resulted in a shift from preferential Th2 response to predominant Th1 responses with remarkable expansion of IFN-γ-producing allergen-specific T-cell clones and strong reduction (p < 0.0001) of IL-4-producing clones with Th2 profile.

### 1.6. HP-NAP shifts the development of bystander activated T cells from Th2 to Th1 phenotype

*In vitro* stimulation with mite allergen also resulted in the expansion of T-cell clones with undefined specificity and unable to proliferate upon re-challenge with mite allergen. In the series of clones with unknown specificity generated from the lines conditioned with medium, none expressed a Th1 profile, 20% were Th0, and 80% were Th2. In contrast, in the corresponding series of clones from the lines conditioned with HP-NAP, 47% were Th1, 37% were Th0 and only 16% were Th2. Of the CD4⁺ clones with unknown specificity derived from the T-cell lines conditioned with IL-12, 28% were Th1, 38% were Th0, and 34% were Th2. Therefore, like for the allergen-specific T-cell response, conditioning with either HP-NAP or IL-12 resulted in significant reduction (p < 0.0001 and p < 0.005, respectively) of Th2 clones and shifting to Th1 and Th0 of the development of the cytokine profile of clones with undefined specificity growing in the context of allergen-induced T-cell lines.

The capacity of HP-NAP to shift to Th1 profile antigen-induced human T-cell responses was confirmed in series of T-cell clones derived from tetanus toxoid-induced T-cell lines conditioned with medium or HP-NAP (Figure 2).

### 1.7. Addition in culture of HP-NAP results in preferential development of cytotoxic T cells

Since cytolytic activity is a common property of activated Th1 and of most Th0, but usually missing in Th2 clones, the cytolytic potential of Th clones was assessed in a lectin (PHA)-dependent ⁵¹Cr-release assay with P815 cells as targets. At an effector to target ratio of 5:1, all the Th0, but none of the Th2 clones generated from allergen-induced T-cell lines conditioned with medium showed cytolytic activity (Figure 3). The mean (±SD) percent specific ⁵¹Cr-release induced by these Th0 clones was low (16.5 ±11). In the series of Th clones derived from T cell lines conditioned with HP-NAP, the few Th2 clones were not cytolytic, whereas all the Th0 and all the Th1 clones expressed cytolytic activity with a significantly higher specific ⁵¹Cr-release in comparison to that of clones from lines conditioned with medium (52 ±21, p<0.0001). Significantly higher ⁵¹Cr-release was still detectable at an effector to target ratio of 1:1 (28 ±15, p<0.0001), suggesting that the cytolytic potential of those clones was high. Like conditioning with HP-NAP, addition of IL-12 resulted in the outgrowth of allergen-specific clones, the majority of which showed cytolytic activity. In fact, none of the Th2, but all the Th1 and Th0 clones were cytolytic (Figure 3).

### 1.8. HP-NAP shifts the development of activated CD8⁺ T cells bystander in culture from Tc2 to Tc1 phenotype.

*In vitro* stimulation with mite allergen or tetanus toxoid also resulted in the expansion of a number of CD8⁺ T-cell clones with undefined specificity and unable to proliferate upon re-challenge with mite allergen or tetanus toxoid, respecitvely. In the series of the 11 CD8⁺ clones with unknown specificity generated from the lines conditioned with medium, 9% expressed a Tc1 profile, 36% were Tc0, and 55% were Tc2. In contrast, in the corresponding series of 10 CD8⁺ clones from the lines conditioned with HP-NAP, 70% were Tc1, 20% were Tc0 and only 10% were Tc2. Of the 9 CD8⁺ clones with unknown specificity derived from the T-cell lines conditioned with IL-12, 56% were Tc1, 33% were Tc0, and 11% were Tc2. Therefore, like for the allergen- or antigen-specific CD4⁺ T-cell responses, conditioning with either HP-NAP or IL-12 resulted in significant reduction (p < 0.025 and p < 0.05, respectively) of Tc2 clones and shifting to Tc1 and Tc0 of the development of the cytokine profile of CD8⁺ clones with undefined specificity growing in the context of allergen- or tetanus toxoid-induced T-cell lines (Figure 4).

### 1.9. HP-NAP-specific T cells from the H. pylori-induced gastric infiltrates are polarized cytotoxic Th1 cells able to produce TNF-α

In order to assess whether *in vitro* data reported above had any *in vivo* correlate, biopsy specimens of antral mucosa from five *H. pylori*-infected patients were cultured for 7 days in IL-2-conditioned medium in order to preferentially expand the *in vivo* activated T cells present in their gastric inflammatory infiltrates. T cell blasts were recovered and cloned by limiting dilution, as reported by D'Elios M.M. et al. [J. Immunol. 158, 962-967, (1997)]. In a series of 144 CD4⁺ T cell clones obtained from the 5 gastric biopsies, 27 (19%) showed significant proliferation in response to HP-NAP. Upon stimulation with HP-NAP, 100% of HP-NAP-specific gastric clones showed a clear-cut Th1 profile. Of note is also the fact that all the HP-NAP-specific clones produced high amounts of TNF-α (4.2 ±3.3 ng/ml, range 0.75-11.4) and all expressed powerful cytolytic activity.

## Claims

1. Neutrophil Activating Protein from *Helicobacter pylori* (HP-NAP) or immmunologically equivalent fragments thereof for enhancing in humans or animals the Th1-type polarization of, or for redirecting spontaneous and undesired Th0/Th2 type into Th1-type, T-cell protective immune responses induced by allergens, endogenous or exogenous antigens, other than antigens of *Helicobacter pylori.*

2. HP-NAP according to claim 1 for use in an immunotherapy of Th2 mediated disorders.

3. HP-NAP according to claims 1 or 2 for use in immunotherapy protocols in the context of allergen immunotherapy, in enhancing the Th1-mediated protection of a vaccine against pathogens, in cancer immunotherapy or in *ex vivo* conditioning of dendtritic cells (DCs).

4. HP-NAP according to claims 1 for redirecting to Tc1 spontaneous and undesired CD8 Tc0/Tc2 T-cell responses to exogenous or endogenous antigens.

5. HP-NAP according to claims 1 for enhancing low efficiency or poor Th1-type responses induced by conventional immunization protocols or poorly protective spontaneous immune responses.

6. HP-NAP according to claims 5 for enhancing poorly effective spontaneous responses to cancer antigens.

7. HP-NAP according to claims 1 for inducing endogenous IL-12 and IL-23 production and eliciting effective Th1-mediated responses to exogenous or endogenous antigens, including neoplastic antigens.

8. HP-NAP according to any one of claims 1 to 7, wherein its immunologically equivalent fragments are fragments including active polypeptide domains of 20-40 amino acid residues.

9. HP-NAP according to any one of claims 1 to 8, wherein in that HP-NAP protein or its fragments are purified.

10. HP-NAP or immmunologically equivalent fragments thereof for use in an *ex vivo* method for the induction of macrophage/DCs maturation *in vitro*, the enhancement of their antigen-presenting cell (APC) function, and deployment of their Th1-polarizing capacity before their reinfusion into the original donors with the aim of eliciting effective Th1-mediated responses to antigen(s) of pathogens or tumors, including the phase in which macrophage/DCs are treated *in vitro* with NH-NAP protein from *Helicobacter pylori* or with its immunologically equivalent fragments.

11. Immunoadjuvant composition comprising HP-NAP or of immmunologically equivalent fragments thereof for the uses according to any one of claims 1 to 7.

12. Immunoadjuvant composition according to claim 11, wherein the immmunologically equivalent fragments are fragments including active polypeptide domains of 20-40 amino acid residues.

13. Immunoadjuvant composition according to claims 11 or 12, wherein HP-NAP protein or its fragments are purified.

14. Immunoadjuvant composition according to any one of claims 11 to 13, wherein HP-NAP protein or its fragments are administered alone, without other exogenous proteins, to redirect to Th1 ongoing spontaneous pathogenic Th0/Th2 responses to undefined antigens or autoantigens.

15. A vaccine composition comprising (a) one or more antigens with the exception of antigens of *Helicobacter pylori,* (b) one or more pharmaceutically acceptable excipients and optional carriers or other immunoregulatory agents, (c) and, as an immune adjuvant, a therapeutically effective amount of the NAP protein of *Helicobacter pylori* (HP-NAP), or immunologically equivalent fragments thereof for use in a treatment against pathogens, with the exception of treatment of *Helicobacter pylori* infections.

16. A composition according to claim 15, **characterized in that** HP-NAP protein, its fragments and the antigens are independently obtained by isolation and purification from cell culture, or by recombinant DNA technology or by chemical synthesis.

17. A composition according to claim 15, **characterized in that** HP-NAP protein, its fragments and the antigens are purified.

18. A composition according to claim 15, **characterized in that** it comprises a diluent selected among water, saline solutions, glycerol, ethanol, and/or auxiliary substances, such as wetting or emulsifying agents, pH buffering substances.

19. A composition according to claim 15, **characterized in that** it comprises one or more allergens used for allergen immunotherapy.

20. A composition according to claim 15, **characterized in that** it comprises as adjuvant one or more fragments of the entire HP-NAP protein including active polypeptide domains of 20-40 amino acid residues.

21. A composition according to claim 15, **characterized in that** it comprises one or more molecular fusion hybrids coupling HP-NAP and an antigen or an allergen.

22. A composition according to claim 21, **characterized in that** it comprises one or more active polypeptide domains of HP-NAP combined with one or more antigens or allergens into a molecular fusion hybrid.

23. A composition according to any one of claims from 15 to 22, suitable for parenteral, transcutaneous, mucosal or oral administration.

24. A method of preparation of the composition according to any one of claims from 15 to 23, **characterized in that** therapeutically effective amounts of HP-NAP from *Helicobacter pylori* or immmunologically equivalent fragments thereof are mixed with one or more antigens or allergens and with excipients and additives usually appropriate for a vaccine composition.

25. A method according to claim 24, **characterized in that** HP-NAP protein, its fragments, antigens or allergens are independently obtained by isolation and purification from cell culture, or by recombinant DNA technology or by chemical synthesis.

## Patentansprüche

1. Neurophiles Aktivierungsprotein von *Helicobacter Pylori* (HP-NAP) oder immunologisch äquivalente Fragmente davon zur Förderung der Thl-Typ Polarisation von oder zur Umlenkung spontaner und unerwünschter Th0/Th2-Type zu Th1-Type T-Zellen schützende(n) Immunresponses, induziert durch Allergene, endogene oder exogene Antigene, außer Antigene von *Helicobacter Pylori*, bei Menschen oder Tieren.

2. HP-NAP nach Anspruch 1, zur Verwendung bei einer Immuntherapie von Th2 vermittelten Störungen.

3. HP-NAP nach Anspruch 1 oder 2, zur Verwendung bei Immuntherapieprotokollen im Zusammenhang mit Allergenimmuntherapie, beim Fördern des Th1-vermittelten Schutzes eines Impfstoffes gegen Pathogene, bei der Krebs-Immuntherapie oder bei der *ex vivo* Konditionierung von dendritischen Zellen (DCs).

4. HP-NAP nach Anspruch 1, zum Umlenken von spontanen und unerwünschten CD8 Tc0/Tc2 T-Zellen Responses auf exogene oder endogene Antigene, zu Tc1.

5. HP-NAP nach Anspruch 1, zum Fördern von niedrig effizienten oder schwachen Th1-Typ Responses, die durch herkömmliche Immunisierungsprotokolle oder schwach schützende spontane Immunresponses induziert werden.

6. HP-NAP nach Anspruch 5, zum Fördern von schwach effektiven spontanen Responses auf Krebsantigene.

7. HP-NAP nach Anspruch 1, zum Induzieren einer endogenen IL-12 und IL-23 Produktion und zum Hervorrufen effektiver Th1 vermittelter Responses auf exogene oder endogene Antigene, einschließlich neoplastischer Antigene.

8. HP-NAP nach einem der Ansprüche 1 bis 7, wobei seine immunologisch äquivalenten Fragmente Fragmente sind, die aktive Polypeptid-Domänen von 20 bis 40 Aminosäureresten enthalten.

9. HP-NAP nach einem der Ansprüche 1 bis 8, wobei jenes HP-NAP-Protein oder seine Fragmente gereinigt ist/sind.

10. HP-NAP oder immunologisch äquivalente Fragmente davon zur Verwendung bei einem *ex vivo* Verfahren für die Induktion von Makrophage/DCs Maturation in vitro, die Förderung ihrer Antigenpräsentationszellen- (APC-) Funktion und Entwicklung ihrer Th1-Polarisationskapazität vor ihrer Re-Infusion in die originalen Spender mit dem Ziel des hervorrufens von effektiven Th1 vermittelten Responses auf Antigen(e) von Pathogenen oder Tumoren, einschließlich der Phase, in welcher Makrophage/DCs *in vitro* mit HP-NAP-Protein von *Helicobacter Pylori* oder mit seinen immunologisch äquivalenten Fragmenten behandelt werden.

11. Immunadjuvant, enthaltend HP-NAP oder immunologisch äquivalente Fragmente davon für die Verwendungen nach einem der Ansprüche 1 bis 7.

12. Immunadjuvant nach Anspruch 11, wobei die immunologisch äquivalenten Fragmente Fragmente sind, die aktive Polypeptid-Domänen von 20 bis 40 Aminosäureresten enthalten.

13. Immunadjuvant nach Anspruch 11 oder 12, wobei das HP-NAP-Protein oder seine Fragmente gereinigt ist/sind.

14. Immunadjuvant nach einem der Ansprüche 11 bis 13, wobei das HP-NAP-Protein oder seine Fragmente alleine ohne andere exogene Proteine verabreicht wird/werden, um zu Th1 weiter gehende spontane pathogene Th0/Th2 Responses auf undefinierte Antigene oder Autoantigene umzulenken.

15. Impfstoffzusammensetzung, enthaltend (a) ein oder mehrere Antigen(e), mit Ausnahme der Antigene von *Helicobacter Pylori*, (b) einen oder mehrere pharmazeutisch akzeptable Exzipienten oder optionale(n) Träger oder andere immunoregulative Stoffe, und (c), als ein Immunadjuvant, eine therapeutisch wirksame Menge des NAP-Proteins von *Helicobacter Pylori* (HP-NAP) oder von immunologisch äquivalenten Fragmenten davon zur Verwendung bei einer Behandlung gegen Pathogene, mit Ausnahme der Behandlung von *Helicobacter Py*lori-Infektionen.

16. Zusammensetzung nach Anspruch 15, **dadurch gekennzeichnet, dass** das HP-NAP-Protein, seine Fragmente und die Antigene unabhängig durch Isolation und Reinigung von Zellkulturen oder durch rekombinante DNA-Technologie oder durch chemische Synthese erhalten werden.

17. Zusammensetzung nach Anspruch 15, **dadurch gekennzeichnet, dass** das HP-NAP-Protein, seine Fragmente und die Antigene gereinigt sind.

18. Zusammensetzung nach Anspruch 15, **dadurch gekennzeichnet, dass** sie ein Verdünnungsmittel enthält, das ausgewählt ist aus Wasser, Salzlösungen, Glycerol, Ethanol und/oder Hilfssubstanzen, wie Befeuchtungs- oder Emulgierungsmitteln, pH-Puffersubstanzen.

19. Zusammensetzung nach Anspruch 15, **dadurch gekennzeichnet, dass** sie ein oder mehrere Allergen(e) enthält, das/die zur Allergenimmuntherapie verwendet wird/werden.

20. Zusammensetzung nach Anspruch 15, **dadurch gekennzeichnet, dass** sie als Adjuvant ein oder mehrere Fragment(e) des gesamten HP-NAP Proteins enthält, einschließlich aktive Polypeptid-Domänen von 20 bis 40 Aminosäureresten.

21. Zusammensetzung nach Anspruch 15, **dadurch gekennzeichnet, dass** sie ein oder mehrere HP-NAP koppelnde Molekularfusionshybrid(e) und ein Antigen oder ein Allergen enthält.

22. Zusammensetzung nach Anspruch 21, **dadurch gekennzeichnet, dass** sie eine oder mehrere aktive Polypeptid-Domäne(n) von HP-NAP kombiniert mit einem oder mehreren Antigen(en) oder Allergen(en) zu einem Molekularfusionshybrid enthält.

23. Zusammensetzung nach einem der Ansprüche 15 bis 22, geeignet zur parenteralen, transcutanen, mucosalen oder oralen Verabreichung.

24. Verfahren zur Bereitstellung der Zusammensetzung nach einem der Ansprüche 15 bis 23, **dadurch gekennzeichnet, dass** therapeutisch effektive Mengen von HP-NAP von *Helicobacter Pylori* oder immunologisch äquivalenten Fragmenten davon mit einem oder mehreren Antigen(en) oder Allergen(en) und mit Hilfsstoffen und Zusätzen gemischt werden, die üblicherweise für eine Impfstoffzusammensetzung geeignet sind.

25. Verfahren nach Anspruch 24, **dadurch gekennzeichnet, dass** HP-NAP Protein, sein Fragmente, Antigene oder Allergene unabhängig durch Isolation und Reinigung von Zellkulturen oder durch rekombinante DNA-Technologie oder durch chemische Synthese erhalten werden.

## Revendications

1. Protéine d'activation des neutrophiles de l'*Helicobater pylori* (HP-NAP) ou portions de celle-ci immunologiquement équivalentes pour augmenter chez les humains ou les animaux la polarisation de type Th1, ou réorienter les types Th0-Th2 spontanés et non désirés en type Th1, des réponses immunes protectrices des cellules T induites par des allergènes, des antigènes endogènes ou exogènes, autres que des antigènes de l'*Helicobacter pylori*.

2. La HP-NAP selon la revendication 1 pour utilisation dans une immunothérapie de désordres médiés par Th2.

3. La HP-NAP selon l'une des revendications 1 et 2 pour utilisation dans des protocoles d'immunothérapie dans le contexte d'immunothérapie par allergènes, dans l'augmentation de la protection médiée par Th1 d'un vaccin contre des pathogènes, dans l'immunothérapie des cancers ou dans le conditionnement *ex vivo* de cellules dendritiques (DCs).

4. La HP-NAP selon la revendication 1 pour réorienter en Tc1 des réponses de cellules T CD8 de type Tc0/Tc2 spontanées et non désirées à des antigènes exogènes ou endogènes.

5. La HP-NAP selon la revendication 1 pour augmenter des réponses efficacement faibles ou pauvres de type Th1 induites par des protocoles d'immunisation conventionnels ou des réponses immunes spontanées pauvrement protectrices.

6. La HP-NAP selon la revendication 5 pour augmenter des réponses spontanées pauvrement efficaces à des antigènes de cancer.

7. La HP-NAP selon la revendication 1 pour induire la production de IL-12 et IL-23 endogènes et obtenir des réponses efficaces médiées par Th1 à des antigènes exogènes ou endogènes, incluant des antigènes néoplastiques.

8. La HP-NAP selon l'une quelconque des revendications 1 à 7, dans laquelle ses portions immunologiquement équivalentes sont des portions comprenant des domaines polypeptidiques actifs de 20-40 résidus d'acides aminés.

9. La HP-NAP selon l'une quelconque des revendications 1 à 8, dans laquelle la protéine HP-NAP ou ses portions sont purifiées.

10. La HP-NAP ou les portions immunologiquement équivalentes de celle-ci pour utilisation dans un procédé *ex vivo* pour l'induction de la maturation *in vitro* de macrophages/DC, l'augmentation de leur fonction de cellules présentatrices d'antigènes (APC), et l'élargissement de leur capacité de polarisation Th1 avant leur ré-injection dans les donneurs d'origine avec le but d'obtenir des réponses efficaces médiées par Th1 à au moins un antigène de pathogènes ou de tumeurs, comprenant la phase dans laquelle les macrophages/DC sont traités *in vitro* avec la protéine NH-NAP de l'*Helicobacter pylori* ou avec ses portions immunologiquement équivalentes.

11. Composition immuno-adjuvante comprenant la HP-NAP ou des portions immunologiquement équivalentes de celle-ci pour des utilisations selon l'une quelconque des revendications 1 à 7.

12. Composition immuno-adjuvante selon la revendication 11, dans laquelle les portions immunologiquement équivalentes sont des portions comprenant des domaines polypeptidiques actifs de 20-40 résidus d'acides aminés.

13. Composition immuno-adjuvante selon l'une des revendications 11 et 12, dans laquelle la protéine HP-NAP ou ses portions sont purifiées.

14. Composition immuno-adjuvante selon l'une des revendications 11 à 13, dans laquelle la protéine HP-NAP ou ses portions sont administrées seules, sans autres protéines exogènes, pour ré-orienter en Th1 des réponses Th0/Th2 habituelles pathogéniques spontanées à des antigènes ou auto-antigènes non définis.

15. Une composition vaccinique comprenant (a) au moins un antigène à l'exception d'antigènes de l'*Helicobacter pylori,* (b) au moins un excipient pharmaceutiquement acceptable et des porteurs optionnels ou autres agents immuno-régulateurs, et (c), à titre d'adjuvant immunitaire, une quantité thérapeutiquement efficace de protéine NAP de l'*Helicobater pylori* (HP-NAP), ou des portions immunologiquement équivalentes de celle-ci pour utilisation dans un traitement contre des pathogènes, à l'exception de traitement d'infections par *Helicobacter pylori*.

16. Une composition selon la revendication 15, **caractérisée en ce que** la protéine HP-NAP, ses portions et les antigènes sont obtenus indépendamment en isolant et purifiant une culture de cellules, ou par technologie de l'ADN recombinant ou par synthèse chimique.

17. Une composition selon la revendication 15, **caractérisée en ce que** la protéine HP-NAP, ses portions et les antigènes sont purifiés.

18. Une composition selon la revendication 15, **caractérisée en ce qu'**elle comprend un diluant choisi parmi l'eau, des solutions salines, le glycérol, l'éthanol, et/ou des substances auxiliaires, telles que des agents mouillants ou émulsifiants, des substances tampons pH.

19. Une composition selon la revendication 15, **caractérisée en ce qu'**elle comprend au moins un allergène utilisé pour l'immunothérapie allergénique.

20. Une composition selon la revendication 15, **caractérisée en ce qu'**elle comprend, comme adjuvant, au moins une portion de la protéine HP-NAP entière incluant des domaines polypeptidiques actifs de 20-40 résidus d'acides aminés.

21. Une composition selon la revendication 15, **caractérisée en ce qu'**elle comprend au moins un hybride par fusion moléculaire couplant la HP-NAP et un antigène ou un allergène.

22. Une composition selon la revendication 21, **caractérisée en ce qu'**elle comprend au moins un domaine polypeptidique actif de la HP-NAP combiné avec au moins un antigène ou un allergène en un hybride par fusion moléculaire.

23. Une composition selon l'une quelconque des revendications 15 à 22, adaptée pour administration parentérale, transcutanée, mucosale ou orale.

24. Un procédé de préparation de la composition selon l'une quelconque des revendications 15 à 23, **caractérisé en ce que** les quantités thérapeutiquement efficaces de la HP-NAP à partir de l'*Helicobacter pylori* ou des portions immunologiquement équivalentes de celle-ci sont mélangées avec au moins un antigène ou un allergène et avec des excipients et des additifs habituellement appropriés pour une composition vaccinique.

25. Un procédé selon la revendication 24, **caractérisé en ce que** la protéine HP-NAP, ses portions, les antigènes ou les allergènes sont obtenus indépendamment en isolant et purifiant une culture de cellules, ou par la technologie de l'ADN recombinant ou par synthèse chimique.
